# EUROPEAN PATENT SPECIFICATION

(11) **EP 0 670 496 B1**
(45) Date of publication and mention of the grant of the patent: **04.10.1995**
(21) Application number: 94102793.0
(22) Date of filing: 24.02.1994
(51) Int. Cl.: G01N 33/58, C12Q 1/42, G01N 1/30

(54) **Double dark-field staining method, stained specimen, and use of enzymatic tracers in double dark-field staining**
Doppeldunkelfeld-Anfärbeverfahren; Proben, erhalten durch das Verfahren und Verwendung von Enzymmarkern in Doppeldunkelfeldfärbungen
Procédé de coloration à double fond obscur, échantillon coloré et utilisation de traceurs enzymatiques dans la coloration à double fond obscur

(43) Date of publication of application: 06.09.1995
(73) Proprietor: Becton, Dickinson and Company, Franklin Lakes, New Jersey 07417-1880 (US)
(72) Inventor: van der Loos, Chris, NL-1317 BJ Almere (NL); Becker, Anton E., NL-1473 PP Warder (NL)
(74) Representative: Frohwitter, Bernhard, Dipl.-Ing.

(56) References cited:
- EP-A- 0 369 362
- JOURNAL OF IMMUNOLOGICAL METHODS., vol.117, 1989, NEW YORK US pages 45 - 52 C. M. VAN DER LOOS ET AL 'MULTIPLE IMMUNOENZYME STAINING TECHNIQUES. USE OF FLUORESCEINATED, BIOTINYLATED AND UNLABELLED MONOCLONAL ANTIBODIES.'
- THE JOURNAL OF HISTOCHEMISTRY AND CYTOCHEMISTRY, vol.40, no.9, 1992 pages 1299 - 1308 E. J. M. SPEEL ET AL. 'A NOVEL FLUORESCENCE DETECTION METHOD FOR IN SITU HYBRIDIZATION, BASED ON THE ALKALINE PHOSPHATASE-FAST RED REACTION.'
- HYSTOCHEMISTRY, vol.71, 1981 pages 513 - 520 K. YAMADA ET AL. 'A DUAL STAINING METHOD FOR NEUTRAL COMPLEX CARBOHYDRATES USING AKALINE PHOSPHATASE-LABELED CONCANAVALIN A AND PERIODIC ACID-SCHIFF.'

## Description

The invention relates to a method for double staining, particularly for light-microscopically analyzing a specimen, and to specimen produced by this method. The invention also relates to the use of enzymatic tracers in double dark-field microscopical methods.

Double staining techniques have been developed in order to identify two or more different components, such as antigens, simultaneously and are a widely used for example in the field of cell biology.

Immunoenzyme double staining methods using two different enzyme tracers, one for each of the two components to be detected, have been shown to be suitable when both components, here antigens, occur at about the same concentration. In this case both single colors at sites of co-localization are simply and reliably distinguished. However, when both antigens occur in concentrations which are widely apart, subtle mixed-colors which result from the precipitates produced by the enzymes may pass unnoticed easily.

Double immunofluorescence methods may yield better results because the use of traditional fluorochromes allows a separate identification of two antigens. However, these methods generally suffer from the disadvantage that immunofluorescence does not provide full microscopic detail and the methods cannot be applied with all tissues and with all types of fixatives. In addition, fluorescence rapidly fades.

Another method for double fluorescence staining is based on the combination of fluorescein isothiocyanate (FITC) as reporter molecule with alkaline phosphatase (AP) activity detection. AP activity when detected with Naphthol-AS-MX-phosphate/Fast Red TR (N-ASMX-P/FRTR) produces a red reaction product which shows a red fluorescence when observed with a fluorescence microscope equipped with a rhodamine filter set. However, it is apparent that this method has the same drawbacks as double immunofluorescence using two traditional fluorochromes, even though the red fluorescence of the N-ASMX-P/FRTR reaction product shows hardly any fading.

In an alternative approach for double staining, in order to improve discrimination between basic colors, the combination of an immunoenzymatic technique and the immunogold/silver staining (IGSS) technique has been used. This opens the possibility to observe both reaction products with bright-field microscopy or visualize silver enhanced gold particles with dark-field epipolarization microscopy. However, separate observation of the two reaction products is not possible and separate black/white photographic recording cannot be done.

Surprisingly it has been found according to the invention that a staining technique producing a fluorescence signal can be combined with a staining technique producing a polarization signal to form a double dark-field method where the signals for the different components do not interfere with each other. This allows separate observation of two antigens with full microscopic detail.

In accordance with this finding a method for double dark-field staining is provided as it is defined in the claims. Furthermore a specimen produced by the method of this invention and as defined in a claim is also provided. In still another aspect of the present invention the use of a tracer enzyme in the double dark-field method of this invention and as defined in a claim is provided. Further embodiments of the invention are also defined in the claims.

Thus, in accordance with the preferred embodiments of this invention for a double dark-field staining method it has been found that a simple and reliable staining technique producing the fluorescence signal is an enzymatical technique where the enzyme involved in producing the fluorescence signal is specifically bound to at least one first component to be detected via one or more antibodies. It is, however, contemplated that the enzyme can also be directly or indirectly bound to other binding partners specific for said first component.

The preferred enzyme is an alkaline phosphatase (AP). In accordance with this embodiment of the invention, an inhibitor of endogenous AP is included in the staining reaction in order to improve the signal/noise ratio of the specimen. By way of example, such a suitable inhibitor is levamizole. In order to prevent a general non-specific background the visualization of AP activity is performed at room temperature for less than five minutes, preferably for two to four minutes.

When a rhodamine filter set is used in a preferred embodiment of the invention the green light excitation is reduced by a grey-filter (25 % transmission). This results in an acceptable decrease of fluorescence intensity while the fading of the fluorescence signal is reduced to a minimum.

In one most preferred embodiment at least one second component is detected by a polarization signal produced by binding of ultra-small gold particles to said component and subsequent visualization of the complex. The gold particles are preferably directly or indirectly bound to said component via specific binding partners such as antibodies.

To improve the visualization of the ultra-small gold particles, a silver enhancement is preferably performed after the staining which produces the fluorescence signal is finished. A possible interference of the silver precipitates with the staining reaction for the at least first component is thus excluded.

In one most preferred embodiment of this invention the specimen are sections such as cryostat of paraffin sections from biological material. Preferably, the biological material comprises cellular objects which are stained for at least two different components, i.e. antigens. The detection of the components in the stained specimen can be performed with standard microscopical techniques.

These and other details and advantages of the invention will become apparent from the following example, the appended claims and the drawings which show in
- FIG. 1: A paraffin section of coronary artery with atherosclerotic changes, double stained for smooth muscle cells (1A4 antibody) and macrophages (HAM56 antibody) according to "protocol d" of the Example.
- FIG.1A: The adventitia contains a small muscular artery with staining of smooth muscle cells of the media in red (AP-fluorescent signal) and endothelial cells and adjacent macrophages in blue (IGSS signal).
- FIG.1B: Detail of the atherosclerotic lesion showing a mixed pattern of smooth muscle cells in red and macrophages in blue.
- FIG. 2: Detail of a branching intramyocardial artery and adjacent myocardial cells showing ischemic changes. Double staining of a cryostat section according to "protocol c" of the Example with two endothelial cell markers: anti-EN4 and anti-PAL-E. PAL-E in red (AP-fluorescent signal) only stains some of the endothelial cells of the artery, whereas EN4 in blue (IGSS signal) stains endothelial cells of the artery and the capillaries.
- FIG. 3: Detail of myocardium. Double staining of a paraffin section according to "protocol a" of the Example with anti-muscle actin (HHF35 antibody) and anti-collagen type IV. Muscle actin in red (AP-fluorescent signal) stains the contractile fibers and intercalated discs. Anti-collagen type IV in blue (IGSS signal) stains the basal membrane of myocytes and capillaries.

The above three figures are illustrations of immuno double staining with a red AP-fluorescence signal and a blue IGSS epipolarization signal. Each horizontal row shows the same tissue sites with a rhodamine filter set (1st column), an epipolarization filter (2nd column), and double exposures showing both signals (3rd column).

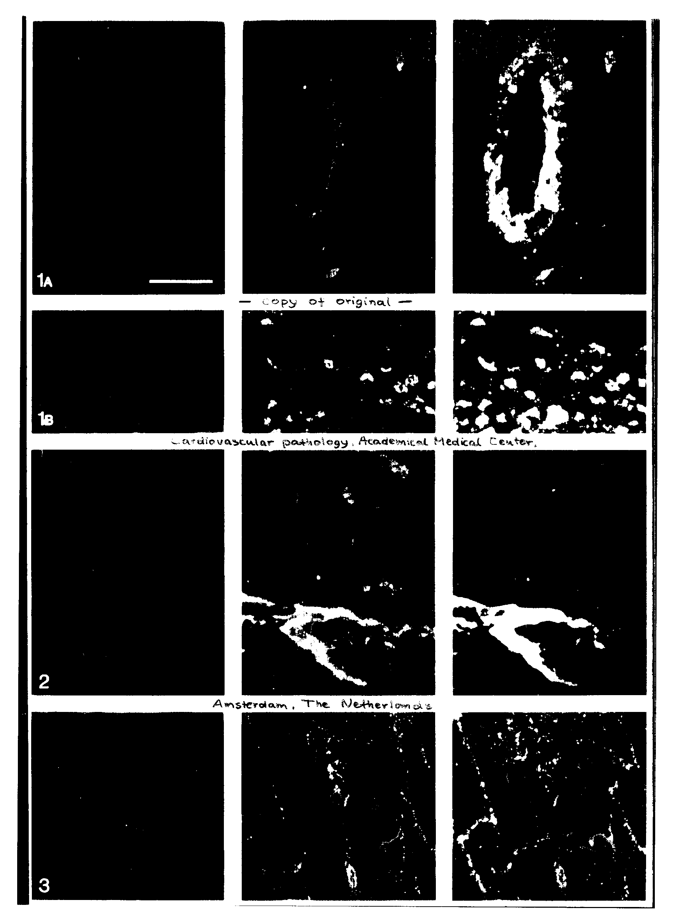

Bar in Fig. 1A = 32µm (accounts for all figures)
The following antibodies and related reagents and the following tissues are useful to reduce the invention to pratice:

### Antibodies and related reagents

Normal goat serum (NGS), normal mouse serum (NMS), rabbit anti-FITC, mouse antimacrophage (clone HAM56, IgM isotype), mouse anti-smooth muscle actin (clone 1A4, IgG2a isotype), alkaline phosphatase conjugated goat anti-mouse Ig (GAM/AP), alkaline phosphatase conjugated goat anti-rabbit Ig (GAR/AP), biotinylated goat anti-mouse Ig(GAM/bio), alkaline phosphatase conjugated streptavidin (Strep/AP), New Fuchsin alkaline phosphatase detection kit and glycerin/gelatin were from DAKO (Glostrup, Denmark). Mouse anti-muscle actin (clone HHF35, IgG1 isotype) was from Enzo Diagnostics (New York, USA). Mouse anti-endothelium antibodies PAL-E (IgG2a isotype) and EN4 (IgG1 isotype) were from MonoSan (Uden, The Netherlands). FITC-conjugated mouse anti-Leu 3a (CD4/FITC), mouse anti-Leu 2a (CD8) and CAS Red alkaline phosphatase detection kit were from Becton & Dickinson (Mountain View, CA USA). Rabbit anti-collagen type IV was from Organon Teknika (Oss, The Netherlands). Ultra small gold labeled goat anti-rabbit Ig (GAR/USG), ultra small gold labeled goat anti-mouse Ig (GAM/USG), ultra small gold labeled goat anti-biotin (GABIO/USG), cold water fish skin gelatin and silver enhancement kit, were from Aurion (Wageningen, The Netherlands). Alkaline phosphatase conjugated goat anti-mouse IgG2a (GAM-IgG2a/AP), biotinylated goat anti-mouse IgM (GAM-IgM/bio) and biotinylated goat anti-mouse IgG1 (GAM-IgG1/bio) were from Southern Biotechnology Associates (Birmingham AL, USA). Naphthol-AS-MX-phosphate (N-ASMX-P), Naphthol-ASBI-phosphate (N-ASBI-P), Fast Red TR, Fast Red Violet LB, polyvinyl alcohol (MW (80,000 - 100,000 hot water soluble) and levamizole were from Sigma (St. Louis. MO USA). New Fuchsin and sodium nitrite were from Merck (Darmstadt, Germany).

### Tissues

Hyperplastic human tonsils were obtained at surgery. Human heart tissue and coronary arteries were obtained at autopsy. The heart specimens showed both normal and ischemic areas. The coronary artery segments contained artherosclerotic lesions with smooth muscle cells and macrophages. Normal human thymus tissue was obtained at autopsy of a 3 months old infant.

Tonsil, thymus and heart tissues were snap frozen in isopentane, cooled with liquid nitrogen. Cryostat sections were cut at 5 µm thickness, mounted on organosilan coated object-glasses, dried overnight, fixed in pure acetone for 10 min at 4°C, wrapped in aluminum-foil and stored at -20°C. The coronary arteries were routinely fixed in 4 % buffered formalin and paraffin embedded. Sections were cut at 5 µm thickness, mounted on organosilan coated object-glasses and dried overnight at 37°C. Before immunohistochemistry the section were dewaxed in xylene and rehydrated in graded alcohols.

Antisera, antibody/enzyme conjugates and streptavidin reagents were diluted in Tris/HCl (50 mM, pH 7.8) buffered saline (TBS) + 1% Bovine Serum Albumin (BSA). TBS washings were performed between all steps (3 x 2 min) and all incubations were performed at room temperature. Antibody/Ultra Small Gold (USG) conjugates were diluted in TBS + 0.8% BSA + 0.1% cold water fish skin gelatin. This solution was also used for washing prior to, and after the incubation with the antibody/ USG conjugate (3 x 10 min).

### EXAMPLE

### Alkaline phosphatase cytochemistry

Before starting with the double staining experiments, the optimal dilution of GAM/AP conjugate was tested. Three different commonly applied AP visualization methods and three commercially available kits, all with a final red reaction product, were compared with respect to their properties in bright-field-, fluorescence- and epipolarization microscopy. Briefly, acetone fixed cryostat sections of the tonsil were subsequently incubated with NGS (15 min), anti-Leu 14 (undiluted, 60 min) and GAM/AP (1:10 - 1:500, 30 min). A negative control consisted of an experiment with omission of the primary antibody. AP activity detection was performed with and without the addition of 9% polyvinyl alcohol (PVA) (Speel et al.).
I Fast Red TR (1 mg/ML) / N-ASMX-P (0.2 mg/ml), according to Speel et al.
II Fast Red Violet LB (0.2 mg/ml) / N-ASMX-P (0.2 mg/ml), according to Van der Loos et al.
III New Fuchsin/N-ASBI-P, according to the DAKO laboratory manual.
IV CAS Red Kit, according to the instructions of the manufacturer (Becton & Dickinson).
V Fast Red Kit, according to the instructions of the manufacturer (BioGenex).
VI New Fuchsin Kit, according to the instructions of the manufacturer (DAKO).

All AP activity visualizations included the addition of 1 mM levamizole and all were performed at room temperature for either 2-4 min ("short") or 7-10 min ("long") (see Table 1). The reaction process was stopped by rinsing the specimen in distilled water. The results were examined with an Olympus BHS photomicroscope, equipped with a mercury lamp (100 W) and a 40x emersion-oil objective. Both a rhodamine-filter set and an epipolarization filter were used.

**Table 1**

| **Bright-field-, fluorescence- and epipolarization microscopy of six alkaline phosphatase reactions in red** | | | | | | |
|---|---|---|---|---|---|---|
| | I | II | III | IV | V | VI |
| "short" | + + (-) | + + + (-) | + + + (-) | + (-) | + + (-) | + + (-) |
| "long" | + + + (±) | + + + + (±) | + + + + (-) | + + (-) | + + + (-) | + + + (±) |
| bright-field microscopy | | | | | | |
| "short" | + + (±) | + + + (-) | + + (+ +) | + + + (-) | + + + (-) | + + (+) |
| "long" | + + + (+ +) | + + + (+) | + + + (+ + +) | + + + (±) | + + + (±) | + + (+ +) |
| fluorescence microscopy with rhodamine filter set | | | | | | |
| "short" | + + + + (+) | + (-) | -(-) | -(-) | + + (-) | -(-) |
| "long" | + + + + (+) | + (-) | -(-) | -(-) | + + (±) | -(-) |
| epipolarization microscopy | | | | | | |
| Anti-Leu 14 was used as primary antibody and GAM/AP for detection. - no staining; ± weak staining; + moderate staining; + + strong staining; + + + + very strong staining. Results are given for "short" (2 to 4 min) and "long" (7 to 10 min) AP activity visualization reaction times. The results of the control experiments, with omission of the primary antibody, are given between brackets. I = Fast Red TR; II = Fast Red Violet LB; III = New Fuchsin; IV = CAS Red Kit (Becton & Dickinson); V = Fast Red Kit (BioGenex); VI = New Fuchsin Kit (DAKO) | | | | | | |

### Double staining protocols

Four different double staining protocols were followed to test the applicability of the AP/IGSS dark-field method of the present invention. Sections were rinsed briefly in TBS and covered with NGS 10% for 15 min. After blotting off the NGS, incubation with the first antibody was started without TBS washing.

| Protocol a: Tested on the paraffin sections of human heart: | |
|---|---|
| 1. Anti-muscle actin (HHF35), mouse (1:20) + Anti-collagen IV, rabbit (1:400) (as a mixture) | 60 min |
| 2. GAR/USG (1:50) | 120 min |
| 3. GAM/bio (1:200) | 30 min |
| 4. Strep/AP (1:50) | 30 min |

| Protocol b: Tested on the cryostat sections of human thymus: | |
|---|---|
| 1. Anti-CD8 (Leu 2a), mouse (1:20) | 60 min |
| 2. GAM/USG (1:50) | 120 min |
| 3. NMS (1:10) | 15 min |
| 4. Anti-CD4/FITC (Leu 3a/FITC), mouse (1:50) | 60 min |
| 5. Anti-FITC, rabbit (1:1000) | 15 min |
| 6. GAR/AP (1:50) | 30 min |

| Protocol c: Tested on the cryostat sections of human ischemic heart: | |
|---|---|
| 1. Anti-EN4, mouse IgG1 (1:20) + Anti-PAL-E, mouse IgG2a (1:10) (as a mixture) | 60 min |
| 2. GAM-IgG/bio (1:100) + GAM-IgG2a/AP (1:50) (as a mixture) | 30 min |
| 3. GABIO/USG (1:50) | 120 min |

| Protocol d: Tested on the paraffin sections of human atherosclerotic coronary artery: | |
|---|---|
| 1. Anti-smooth muscle actin (1A4), mouse IgG2a (1:100) + anti-macrophage (HAM56), mouse IgM (1:50) (as a mixture) | 60 min |
| 2. GAM-IgG2a/AP (1:50) + GAM-IgM/bio (1:100) (as a mixture) | 30 min |
| 3. GABIO/USG (1:50) | 120 min |

After performance of all steps, AP activity was detected first. Subsequently, the sections were thoroughly rinsed with running tap water (15 min) and cleansed with distilled water (5 min). Next, silver enhancement was performed according to the instructions of the manufacturer (Aurion), at room temperature for 6 - 10 min. After washing with distilled water the sections were mounted with glycerin/gelatin. Some specimens were counterstained with hematoxylin after silver enhancement. Fluorescence and epipolarization signals were recorded using HP5 (Ilford) for black/white and Ektachrome EL-400 (Kodak) for color photography. Both 400 ASA films were exposed at 3200 ASA, but routinely processed.

### Results

Titration of the GAM/AP conjugate showed a weak red staining at a dilution of 1:50 and a "short" AP activity detection when observed with bright-field microscopy. With the rhodamine filter set an intensely red fluorescence signal was obtained with green light excitation. At a lower dilution of the GAM/AP conjugate a gradual increase of the red precipitate occurred with bright-field illumination, but there was no increase of the fluorescence signal intensity. Therefore, the optimal 1:50 dilution for GAM/AP and GAR/AP was used throughout the study. A short nuclear counterstain with hematoxylin did not interfere with the double staining results.

With single staining, using the six AP activity visualization techniques, a moderate (chromogens III and VI) to strong (chromogen I, II, IV and V) red fluorescent precipitate was obtained (Table 1). Three of those (chromogens I, II and V) also showed moderate to high signal levels with epipolarization illumination, with images very close to those of silver enhanced gold particles. Only the red reaction product obtained with the CAS Red kit was completely dark when observed with epipolarization microscopy. A "long" AP activity reaction time gave a stronger bright-field signal, but it did not appear as a stronger fluorescence signal irrespective of the AP method applied. Specimens undergoing "long" AP activity reaction times, showed a relatively high non-specific fluorescence of all tissue elements compared to "short" reactions. The New Fuchsin methods (chromogens III and VI) produced high non-specific background fluorescence, despite the fact that bright-field microscopy yielded distinct images of the precipitate. The addition of 9% PVA marginally improved the sharpness of the precipitate localization, but it did not influence the characteristics of the precipitates with respect to epipolarization microscopy. On the other hand, when performing single IGSS (even up to an intense black bright-field signal) no signal was observed upon green light excitation.

The results are illustrated in Figures 1 - 3, showing black/white or color photographic recording of fluorescence- and epipolarization microscopy. Double exposures (third column) show examples of separate and of overlapping staining patterns. In the latter circumstance, sites of co-localization are distinguished readily from both basic colors (Figure 2).

The histological results of the CD4/CD8 double staining on the thymus using protocol b, was according to expectation: immature thymocytes in the cortex expressed double positivity, whereas mature thymocytes in the medulla appeared either single CD4 or CD8 positive (not illustrated).

### Discussion

This invention presents a method for double staining combining immunofluorescence and immunoenzymatic techniques. The method is based on the combination of a commercially available AP activity revealing technique, in which the reaction product shows an intense red fluorescence, but no signal with epipolarization illumination, and the IGSS technique, which produces a distinct signal with epipolarization, but no signal with the fluorescence filter set.

So far it has been reported that the red AP-fluorescence signal does not fade upon green light excitation or storage of the specimen. Speel et al. who studied this aspect in more detail, described that only at highly intense green light exposure, longer than 40 minutes, some fading became apparent. Our observations confirm these data. Using an older type of fluorescence microscope, there was no fading at all, but the same specimen examined with the Olympus BHS fluorescence system showed fading after 10 minutes of green light exposure. Reduction of the green light excitation by a grey-filter (25% transmission) decreased the fluorescence intensity with about a factor 2, and fading was minimal. Specimens which are stored for 10 months at room temperature sheltered from direct light still exhibit bright fluorescence and epipolarization signals.

Despite the fact that the AP/antibody conjugates were diluted 2 to 5 times more for the fluorescent technique than for a bright-filed method, the staining efficiencies are at least similar or maybe even in favor of the fluorescent technique. Since minute amounts of the AP activity reaction product array showed an intense fluorescence, it is generally recommended to stain AP activity until the moment it becomes weakly visible with bright-filed microscopy. Our observation is that a stronger bright-field signal does not yield a more intense fluorescence signal. In order to prevent a general non-specific background fluorescence (Table 1), the AP activity detection should be completed in less than 5 min. This background fluorescence is almost certainly caused by the spontaneous binding of chromogens to tissue components.

The staining efficiency of the IGSS technique is superior, especially when using ultra small gold labeled antibodies. Because of the high sensitivity of epipolarization compared to bright-filed illumination, the ultra small gold particles were silver enhanced just up till the moment that a brownish silver precipitate became visible with bright-filed microscopy.

In the double staining protocols AP activity was visualized first, followed by silver enhancement. However, the order of visualization did not make much difference, at least with the four tested antibody combinations. Because silver enhancement should be performed after removal of all buffer salts, it is most convenient from a practical point of view to perform AP activity visualization first. Another reason to perform silver enhancement last is that a silver precipitate may shelter an antigen or even a complete antibody "tree" during double staining. A similar phenomenon occurs for the diaminobenzidin polymer after the peroxidase reaction. Thus far the sheltering effects of AP chromogens have not been described. Apart from that , it seems highly unlikely that the formation of only discrete amounts of AP reaction product can cause "hiding" of the antigen. In immunoenzyme double staining protocols the question arises which antigen should be stained and in what color; for the present AP/IGSS dark-field technique the choice is less pressing because the final reaction products are identified separately. Only if a precise localization of an antigen is needed, one may consider the more precise IGSS method instead of the AP technique.

For the first time since the description of double-immunofluorescence a double staining technique is described allowing separate observation of the two antigens. Its major advantage over traditional double-fluorescence is the fact that stained specimens can be stored at room temperature for a considerable length of time without spontaneous fading of fluorescence, while bleaching caused by excitation light is minimal. The present double dark-field technique is simple, utilizes commercially available reagents and it compatible with current immunoenzyme double staining protocols.

### Literature:

Speel et al.: A novel fluorescence detection method for in situ hybridization, based on the alkaline phosphatase-Fast Red reaction. J. Histochem. Cytochem. 40: 1299, 1992.
Van der Loos et al.: Multiple immunoenzyme staining techniques. Use of fluoresceinated, biotinylated and unlabelled monoclonal antibodies. J. Imm. Meth. 117: 45, 1989.

## Claims

1. A double dark-field staining method for analyzing fluorescence and polarization signals of a specimen, said method comprising the steps of:
(a) preparing a specimen for subsequent staining;
(b) staining the specimen for at least one first component by a staining technique producing the fluorescence signal, but essentially no polarization signal when the signals are detected by means of a fluorescence filter set and polarization filter, respectively; and
(c) staining the specimen for at least one second component by a staining technique producing the polarization signal, but essentially no fluorescence signal with said fluorescence filter set.

2. The method of claim 1, wherein the polarization filter is an epipolarization filter.

3. The method of claims 1 or 2, wherein the staining technique of step (b) is an enzymatical technique, in particular an immunoenzymatical technique wherein the enzyme involved in producing said signal is bound to said at least one first component via an antibody/antibodies.

4. The method of claim 3, wherein the enzyme is an alkaline phosphatase (AP).

5. The method of claim 4, wherein the staining with AP includes the addition of an inhibitor of endogenous AP, e.g. levamizole, and the visualization of AP activity is performed at room temperature for less than five minutes, in particular for two to four minutes.

6. The method of any of the preceding claims, wherein said fluorescence signal, when using a rhodamine filter set, is a red fluorescence upon green light excitation.

7. The method of claim 6, wherein the green light excitation is reduced by a grey-filter.

8. The method of any of the preceding claims, wherein the staining technique of step (c) comprises binding of ultra-small gold particles to said at least one second component, in particular via an antibody/antibodies, preceding, simultaneous with or subsequent to step (b).

9. The method of claim 8, wherein the ultra-small gold particles are silver-enhanced, in particular until a silver precipitate becomes visible when controlling with bright-field microscopy.

10. The method of claim 9, wherein the silver enhancement included in step (c) is performed after the staining according to step (b) is finished.

11. The method of any of the preceding claims, wherein said specimen are sections, e.g. cryostat or paraffin sections, from biological material comprising in particular cellular objects.

12. A specimen as obtainable by the method of claims 1 - 11 for use in light-microscopical analysis and in quantitation methods.

13. The use of a tracer enzyme, in particular of alkaline phosphatase, in a double dark-field microscopical method according to claims 1 - 11.

## Patentansprüche

1. Doppel-Dunkelfeld-Anfärbungsverfahren zur Analyse von Fluoreszenz- und Polarisationssignalen eines Präparats, wobei das Verfahren die folgenden Schritte umfaßt:
(a) Herstellen eines Präparats für die anschließende Anfärbung;
(b) Anfärben des Präparats für mindestens eine erste Komponente durch ein Anfärbungsverfahren, das das Fluoreszenzsignal erzeugt, aber im wesentlichen kein Polarisationssignal, wenn die Signale mittels eines Fluoreszenzfilter-Satzes bzw. Polarisationsfilters festgestellt werden; und
(c) Anfärben des Präparats für mindestens eine zweite Komponente durch ein Anfärbungsverfahren, das das Polarisationssignal erzeugt, aber im wesentlichen kein Fluoreszenzsignal mit dem Fluoreszenzfilter-Satz.

2. Verfahren nach Anspruch 1, wobei der Polarisationsfilter ein Epipolarisationsfilter ist.

3. Verfahren nach Anspruch 1 oder 2, wobei das Anfärbungsverfahren von Schritt (b) ein enzymatisches Verfahren ist, insbesondere ein immunenzymatisches Verfahren, wobei das Enzym, das an dem Erzeugen des Signals beteiligt ist, an die mindestens eine erste Komponente über einen Antikörper bzw. über Antikörper gebunden ist.

4. Verfahren nach Anspruch 3, wobei das Enzym eine Alkalische Phosphatase (AP) ist.

5. Verfahren nach Anspruch 4, wobei das Anfärben mit AP die Zugabe eines Inhibitors der endogenen AP, z.B. Levamizol, einschließt, und die Sichtbarmachung der AP-Aktivität bei Raumtemperatur für weniger als fünf Minuten, insbesondere für zwei bis vier Minuten, durchgeführt wird.

6. Verfahren nach einem der vorhergehenden Ansprüche, wobei das Fluoreszenzsignal, wenn ein Satz Rhodamin-Filter verwendet wird, eine rote Fluoreszenz nach Anregung mit grünem Licht ist.

7. Verfahren nach Anspruch 6, wobei die Anregung unter Verwendung von grünem Licht durch einen Graufilter verringert wird.

8. Verfahren nach einem der vorhergehenden Ansprüche, wobei das Anfärbungsverfahren von Schritt (c) das Binden von ultra-kleinen Goldpartikeln an die mindestens ein zweite Komponente unfaßt, insbesondere über einen Antikörper bzw. über Antikörper, vorangehend, gleichzeitig oder anschließend an Schritt (b).

9. Verfahren nach Anspruch 8, wobei die ultra-kleinen Goldpartikel Silber-verstärkt sind, insbesondere bis ein Silberpräzipitat sichtbar wird unter Kontrolle mit Hellfeld-Mikroskopie.

10. Verfahren nach Anspruch 9, wobei die in Schritt (c) inbegriffene Silberverstärkung durchgeführt wird nachdem das Anfärben gemäß Schritt (b) beendet ist.

11. Verfahren nach einem der vorhergehenden Ansprüche, wobei die Präparate Schnitte sind, z.B. Cryostat- oder Paraffinschnitte, von biologischem Material, das insbesondere zelluläre Objekte umfaßt.

12. Präparat, wie es durch das Verfahren nach Ansprüchen 1 - 11 erhältlich ist, zur Verwendung in lichtmikroskopischen Analysen und in Quantifizierungsverfahren.

13. Verwendung eines Tracer-Enzyms, insbesondere von Alkalischer Phosphatase, in einem mikroskopischen Doppel-Dunkelfeld-Verfahren gemäß Ansprüchen 1 - 11.

## Revendications

1. Un procédé de double coloration à fond noir pour l'analyse de signaux de fluorescence et de polarisation d'un spécimen, ce procédé comprenant les étapes suivantes :
(a) préparation d'un spécimen pour coloration ultérieure ;
(b) coloration d'au moins un premier constituant du spécimen par une technique de coloration produisant le signal de fluorescence, mais essentiellement aucun signal de polarisation lorsque les signaux sont détectés au moyen d'un jeu de filtres pour fluorescence et d'un filtre de polarisation, respectivement ; et
(c) coloration d'au moins un second constituant du spécimen par une technique de coloration produisant le signal de polarisation, mais essentiellement aucun signal de fluorescence avec le jeu de filtres pour fluorescence.

2. Le procédé de la revendication 1 dans lequel le filtre de polarisation est un filtre d'épipolarisation.

3. Le procédé de la revendication 1 ou 2, dans lequel la technique de coloration de l'étape (b) est une technique enzymatique, en particulier une technique immunoenzymatique dans laquelle l'enzyme impliquée pour produire ledit signal est liée audit premier constituant au moins via un anticorps / des anticorps.

4. Le procédé de la revendication 3, dans lequel l'enzyme est une phosphatase alcaline (AP).

5. Le procédé de la revendication 4, dans lequel la coloration avec l'AP comporte l'addition d'un inhibiteur de l'AP endogène, par exemple le levamizole, et la visualisation de l'activité AP est effectuée à la température de la salle pendant moins de cinq minutes, en particulier pendant deux à quatre minutes.

6. Le procédé de l'une des revendications précédentes, dans lequel le signal de fluorescence, lorsque l'on utilise un jeu de filtres rhodamine, est une fluorescence rouge pour excitation en lumière verte.

7. Le procédé de la revendication 6, où l'excitation lumineuse verte est réduite par un filtre gris.

8. Le procédé de l'une des revendications précédentes, dans lequel la technique de coloration de l'étape (c) comporte la liaison de particules d'or ultrapetites audit second constituant au moins, en particulier via un anticorps / des anticorps, avant, en même temps ou après l'étape (b).

9. Le procédé de la revendication 8, dans lequel les particules d'or ultrapetites sont renforcées à l'argent, en particulier jusqu'à ce qu'un précipité d'argent devienne visible par contrôle en microscopie à fond clair.

10. Le procédé de la revendication 9, dans lequel le renforcement à l'argent compris dans l'étape (c) est effectué après que l'on ait terminé la coloration selon l'étape (d).

11. Le procédé de l'une des revendications précédentes, dans lequel le spécimen est constitué de coupes, par exemple de coupes au cryostat ou à la paraffine, de matière biologique comprenant en particulier des objets cellulaires.

12. Un spécimen susceptible d'être obtenu par le procédé des revendications 1 à 11, pour une utilisation en analyse par microscopie optique et dans des procédés quantitatifs.

13. L'utilisation d'une enzyme traceuse, en particulier d'une phosphatase alcaline, dans un procédé de double microscopie à fond noir selon les revendications 1 à 11.
